# EUROPÄISCHE PATENTSCHRIFT

(11) **EP 0 581 181 B1**
(45) Veröffentlichungstag und Bekanntmachung des Hinweises auf die Patenterteilung: **06.12.1995**
(21) Anmeldenummer: 93111648.7
(22) Anmeldetag: 21.07.1993
(51) Int. Cl.: C07D 231/12, C25B 3/02

(54) **Verfahren zur Herstellung von 4-Dialkoxymethylpyrazolen**
Process for the preparation of 4-dialkoxymetylpyrazoles
Procédé pour la préparation de dérivés de 4-dialkoxyméthylpyrazole

(30) Priorität: 29.07.1992 DE 4225053
(43) Veröffentlichungstag der Anmeldung: 02.02.1994
(73) Patentinhaber: BASF Aktiengesellschaft, 67063 Ludwigshafen (DE)
(72) Erfinder: Hermeling, Dieter, Dr., D-6710 Frankenthal (DE); Koenig, Hartmann, dr., D-6703 Limburgerhof (DE); Goetz, Norbert, Dr., D-6520 Worms 1 (DE)

(56) Entgegenhaltungen:
- EP-A- 0 012 240
- EP-A- 0 287 954
- EP-A- 0 378 755
- US-A- 3 254 093
- CHEMICAL ABSTRACTS, vol. 98, 1983, Columbus, Ohio, US; abstract no. 125960t, "Synthesis of 4-formylpyrazole-3(5)-carboxylic acid derivatives by 1,3-dipolar cycloaddition of diazomethane."
- CHEMICAL ABSTRACTS, vol. 102, 1985, Columbus, Ohio, US; abstract no. 62138j, "1,3-Dipolar cycloadditions to 4,4-dimethoxy-2-butynoic acid derivatives: synthesis of substituted pyrazoles and triazoles.", Seite 591.

## Beschreibung

Die vorliegende Erfindung betrifft ein neues Verfahren zur Herstellung von 4-Dialkoxymethylpyrazolen der allgemeinen Formel I 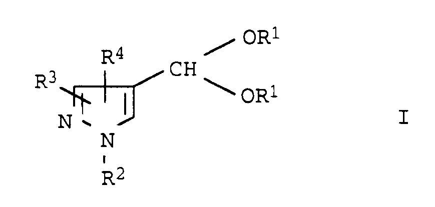
in der die Substituenten folgende Bedeutung haben:
- R¹: n-Alkyl
- R²: C₁-C₁₂-Alkylgruppen, C₃-C₁₂-Cycloalkylgruppen oder Phenylgruppen, die jeweils gegebenenfalls substituiert sein können
- R³, R⁴: Wasserstoff, C₃-C₁₂-Cycloalkylgruppen oder Phenylgruppen, die jeweils gegebenenfalls substituiert sein können, Cyangruppen, Halogen oder Alkoxycarbonylgruppen.

Die Pyrazole I dienen als Zwischenprodukte für die Herstellung von Pflanzenschutzmitteln und Pharmazeutika.

Die Verfahrensprodukte sind zum Teil bekannt. Sie können nach der Lehre der US-A 3,254,093 aus nur sehr aufwendig herstellbaren 1,2,3-Oxadiazolen gewonnen werden.

Die elektrochemische Überführung methylsubstituierter isocyclischer Aromaten in die entsprechenden Acetale wird in der EP-A 012 240 beschrieben.

Es bestand daher die Aufgabe, die Pyrazole I auf einfache Weise aus gut zugänglichen Ausgangsverbindungen herzustellen.

Demgemäß wurde ein Verfahren zur Herstellung der Dialkylmethylpyrazole I gefunden, das dadurch gekennzeichnet ist, daß man ein 4-Methylpyrazol der allgemeinen Formel II 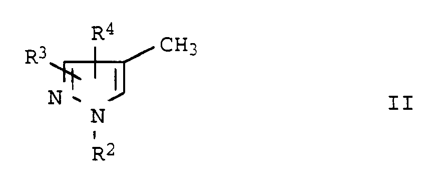
in Gegenwart eines Alkanols R¹-OH elektrochemisch oxidiert.

Das erfindungsgemäße Verfahren kann folgendermaßen veranschaulicht werden: 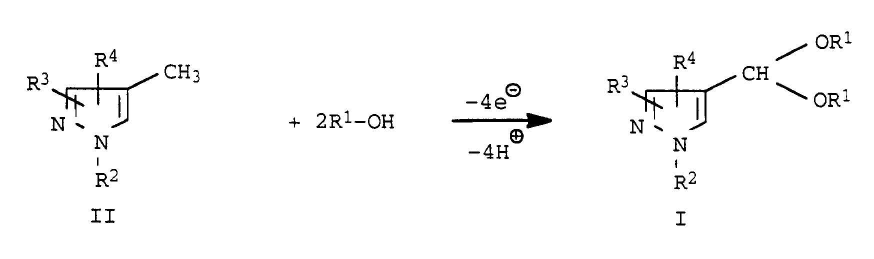
Die Ausgangsverbindungen II sind z. T. bekannt oder können nach bekannten Methoden, wie z.B. durch die Umsetzung von 1,3-Diketoverbindungen mit Hydrazinen (DE-A 29 22 591), hergestellt werden.

Im Hinblick auf ihre Verwendung als Zwischenprodukte für Pflanzenschutzmittel und Pharmazeutika sind unter den Verbindungen I und damit auch unter den Ausgangsverbindungen solche bevorzugt, in denen die Substituenten folgende Bedeutung haben:
- R¹: - C₁-C₆-n-Alkyl, vorzugsweise C₁-C₄-n-Alkyl, vor allem Methyl und Ethyl;
- R²: - C₁-C₁₂-Alkyl, vorzugsweise C₁-C₈-Alkyl, darunter besonders Methyl, Ethyl, n-Propyl, iso-Propyl, n-Butyl, tert.-Butyl, n-Pentyl, tert.-Amyl, n-Hexyl, n-Heptyl, n-Octyl;
- C₃-C₁₂-Cycloalkyl, vorzugsweise C₅-C₇-Cycloalkyl wie Cyclopentyl, Cyclohexyl, Cycloheptyl;
- unsubstituiertes oder substituiertes Phenyl wie Phenyl, 2-Chlorphenyl, 3-Chlorphenyl, 4-Chlorphenyl, 2-Fluorphenyl, 3-Fluorphenyl, 4-Fluorphenyl, 2-Bromphenyl, 3-Bromphenyl, 4-Bromphenyl, 2-Methylphenyl, 3-Methylphenyl, 4-Methylphenyl, 2-Methoxyphenyl, 3-Methoxyphenyl, 4-Methoxyphenyl, 2-Ethoxyphenyl, 3-Ethoxyphenyl, 4-Ethoxyphenyl, 2-tert.-Butoxyphenyl, 3-tert.-Butoxyphenyl, 4-tert.-Butoxyphenyl, 2-Methoxycarbonylphenyl, 3-Methoxycarbonylphenyl, 4-Methoxycarbonylphenyl, 2-Ethoxycarbonylphenyl, 3-Ethoxycarbonylphenyl, 4-Ethoxycarbonylphenyl, 2-Cyanophenyl, 3-Cyanophenyl, 4-Cyanophenyl, 2-Methoxy-4-chlorphenyl, 2,4-Dichlorphenyl, 3-Methyl-6-chlorphenyl, 2,4,6-Trichlorphenyl, 2-N-Formylaminophenyl, 3-N-Formylaminophenyl, 4-N-Formylaminophenyl, 2-N-Carboxymethylaminophenyl, 3-N-Carboxymethylaminophenyl, 4-N-Carboxymethylaminophenyl;
- R³, R⁴: - wie R²; weiterhin:
- Wasserstoff;
- Cyan;
- Halogen wie Fluor, Chlor, Brom, Jod, bevorzugt Fluor, Chlor, Brom;
- Alkoxycarbonyl wie Methoxycarbonyl, Ethoxycarbonyl, tert.-Butoxycarbonyl.

Weiterhin können die Alkyl- und Cycloalkylgruppen in R², R³ und R⁴ Substituenten tragen, wobei C₁-C₄-Alkylreste, C₁-C₄-Alkoxyreste, C₁-C₅-Alkoxycarbonylreste, Hydroxygruppen oder Halogen bevorzugt sind.

Die elektrochemischen Oxidationen können in geteilten, vorzugsweise in ungeteilten Durchflußzellen ausgeführt werden.

Der Elektrolyt setzt sich aus der Ausgangsverbindung II und einem Alkohol R¹-OH zusammen, wobei zur Verbesserung der Leitfähigkeit in einer bevorzugten Ausführungsform ein Hilfselektrolyt zugesetzt wird. Der Elektrolyt kann ein unter den Elektrolysebedingungen inertes Lösungsmittel wie Acetonitril oder Methylenchlorid enthalten; vorzugsweise führt man die Elektrolyse aber ohne ein solches Lösungsmittel durch.

Bevorzugt enthält der Elektrolyt:
1 - 49 Gew.-%, vorzugsweise 5 - 30 Gew.-% des Methylpyrazols II,
50 - 98,9 Gew.-%, vorzugsweise 70 bis 95 Gew.-% des Alkohols R¹OH und
0,1 - 5 Gew.-%, vorzugsweise 0,2 - 3 Gew.-% des Hilfselektrolyten.

Als Hilfselektrolyte kommen Salze, Säuren und Basen in Betracht. Beispiele für Salze sind Fluoride wie Kaliumfluorid, Sulfate wie Tetramethylammoniummethylsulfat, Tetrafluoroborate wie Natriumtetrafluoroborat sowie Phosphate und Phosphonate; bevorzugt wird Natriumbenzolsulfonat.

Beispiele für Säuren sind Schwefelsäure, Alkyl- und Arylsulfonsäuren wie Methylsulfonsäure und Benzolsulfonsäure. Als Basen kommen beispielsweise Alkoholate wie Natriummethylat und Hydroxide wie Kaliumhydroxid in Betracht.

Als Anodenmaterialien kommen Edelmetalle wie Platin oder Oxide wie Chrom- oder Rutheniumoxid sowie Mischoxide wie Ti/RuOₓ in Frage. Bevorzugtes Anodenmaterial ist aber Graphit.

Als Kathodenmaterial eignen sich im allgemeinen Stahl, Eisen, Kupfer, Zinn, Zink, Nickel und Kohle sowie Edelmetalle wie Platin; bevorzugt wird jedoch Graphit.

Die Elektrolyse kann diskontinuierlich oder kontinuierlich ausgeführt werden, wobei nicht umgesetztes Ausgangsmaterial gewünschtenfalls wieder in die Reaktion zurückgeführt werden kann.

Die Stromdichte kann für das erfindungsgemäße Verfahren in weiten Grenzen von 0,1 bis 25 A/dm², vorzugsweise 1 bis 10 A/dm² gewählt werden. Die Oxidationen werden in der Regel bei Temperaturen von 0 bis 120°C, vorzugsweise bei 20 bis 80°C durchgeführt. Dabei herrscht im allgemeinen Normaldruck. Es kann aber auch bei vermindertem Druck oder bei Anwesenheit leicht siedender Komponenten bei erhöhtem Druck gearbeitet werden. Die Ladungsmengen betragen in der Regel 3,5 bis 12, vorzugsweise 4 bis 8 F/mol II.

Die Aufarbeitung der Reaktionslösung erfolgt nach bekannten Methoden, vorzugsweise destillativ.

Das erfindungsgemäße Verfahren überführt die gut zugänglichen 4-Methylpyrazole II in 4-Dialkoxymethylpyrazole I.

Die Acetale I können in an sich bekannter Weise zu den entsprechenden 4-Formylpyrazolen III 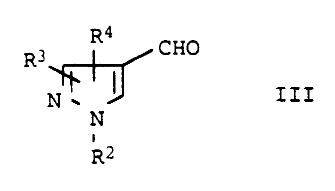
hydrolysiert werden. Die Verbindungen I stellen somit lagerstabile Depotverbindungen für die wesentlich empfindlicheren Aldehyde III dar.

Die 4-Formylpyrazole III dienen als Pflanzenschutzvorprodukte (EP-A 378 755), als Pharmavorprodukte (EP-A 284 914) oder als Vorprodukte für optische Aufheller (DE-A 17 70 614).

### Beispiele

### Beispiel 1

### Elektrosynthese von 4-Dimethoxymethyl-1-phenylpyrazol

- Apparatur:: ungeteilte Zelle mit 11 bipolaren Elektroden
- Anode:: Graphit
- Elektrolyst:: 80 g (506 mmol 4-Methyl-1-phenylpyrazol
30 g Natriumbenzolsulfonat
2,89 kg Methanol
- Kathode:: Graphit
- Elektrolysetemperatur:: 40°C

Die Elektrolyse wurde mit 5 F/mol 4-Methyl-1-phenylpyrazol durchgeführt. Der Elektrolyt wurde während der Elektrolyse mit 200 l/h durch die Zelle geleitet. Nach Beendigung der Elektrolyse wurde das Methanol bei Normaldruck abdestilliert, und das ausgefallene Leitsalz wurde abfiltriert. Nach einer Vakuumdestillation erhielt man bei 86 %igem Umsatz 50 % 4-Dimethoxymethyl-1-phenylpyrazol, was einer Ausbeute bezogen auf umgesetzte Ausgangsverbindung (Selektivität) von 60 % entspricht.

### Beispiel 2

### Elektrosynthese von 1-(4-Chlorphenyl)-4-dimethoxymethylpyrazol

- Apparatur:: ungeteilte Zelle mit 11 bipolaren Elektroden
- Anode:: Graphit
- Elektrolyt:: 68 g (353 mmol) 1-(4-Chlorphenyl)-4-methylpyrazol
30 g Natriumbenzolsulfonat
2,90 kg Methanol
- Kathode:: Graphit
- Elektrolysetemperatur:: 30°C

Die Elektrolyse wurde mit 5,5 F/mol 1-(4-Chlorphenyl)-4-methylpyrazol durchgeführt. Der Elektrolyt wurde während der Elektrolyse mit 200 l/h durch die Zelle geleitet. Nach Durchführung und Aufarbeitung wie in Beispiel 1 wurden 50 % 1-(4-Chlorphenyl)-4-dimethoxymethylpyrazol isoliert (Selektivität 62 %).

### Beispiel 3

### Elektrosynthese von 1-(3-Chlorphenyl)-4-dimethoxymethylpyrazol

- Apparatur:: ungeteilte Zelle mit 9 bipolaren Elektroden
- Anode:: Graphit
- Elektrolyt:: 76 g (395 mmol) 1-(3-Chlorphenyl)-4-methylpyrazol
12 g Natriumbenzolsulfonat
530 g Methanol
- Kathode:: Graphit
- Elektrolysetemperatur:: 25°C

Die Elektrolyse wurde mit 6 F/mol 1-(3-Chlorphenyl)-4-methylpyrazol durchgeführt. Der Elektrolyt wurde während der Elektrolyse mit 20 l/h durch die Zelle geleitet. Nach Durchführung und Aufarbeitung wie in Beispiel 1 wurden 34 % 1-(3-Chlorphenyl)-4-dimethoxymethylpyrazol isoliert (Selektivität 40 %).

### Beispiel 4

### Elektrosynthese von 1-(2-Chlorphenyl)-4-dimethoxymethylpyrazol

- Apparatur:: ungeteilte Zelle mit 9 bipolaren Elektroden
- Anode:: Graphit
- Elektrolyt:: 102 g (530 mmol) 1-(2-Chlorphenyl)-4-methylpyrazol
14 g Natriumbenzolsulfonat
600 g Methanol
- Kathode:: Graphit
- Elektrolysetemperatur:: 20°C

Die Elektrolyse wurde mit 6 F/mol 1-(2-Chlorphenyl)-4-methylpyrazol durchgeführt. Der Elektrolyt wurde während der Elektrolyse mit 20 l/h durch die Zelle geleitet. Nach Durchführung und Aufarbeitung wie in Beispiel 1 wurden 40 % 1-(2-Chlorphenyl)-4-dimethoxymethylpyrazol isoliert (Selektivität 44 %).

### Beispiel 5

### Elektrosynthese von 4-Dimethoxymethyl-1-methylpyrazol

- Apparatur:: ungeteilte Zelle mit 9 bipolaren Elektroden
- Anode:: Graphit
- Elektrolyt:: 100 g (1,04 mol) 1,4-Dimethylpyrazol
16 g Natriumbenzolsulfonat
700 g Methanol
- Kathode:: Graphit
- Elektrolysetemperatur:: 20°C

Die Elektrolyse wurde mit 7 F/mol 1,4-Dimethylpyrazol durchgeführt. Der Elektrolyt wurde während der Elektrolyse mit 20 l/h durch die Zelle geleitet. Nach Durchführung und Aufarbeitung wie in Beispiel 1 wurden 53 % 4-Dimethylpyrazol isoliert (Selektivität 58 %).

## Patentansprüche

1. Verfahren zur Herstellung von 4-Dialkoxymethylpyrazolen der allgemeinen Formel I 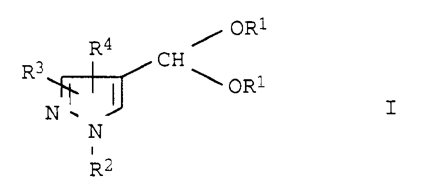 in der die Substituenten folgende Bedeutung haben:
R¹ n-Alkyl
R² C₁-C₁₂-Alkylgruppen, C₃-C₁₂-Cycloalkylgruppen oder Phenylgruppen, die jeweils gegebenenfalls substituiert sein können
R³, R⁴ Wasserstoff, C₃-C₁₂-Cycloalkylgruppen oder Phenylgruppen, die jeweils gegebenenfalls substituiert sein können, Cyangruppen, Halogen oder Alkoxycarbonylgruppen.
dadurch gekennzeichnet, daß man ein 4-Methylpyrazol der allgemeinen Formel II 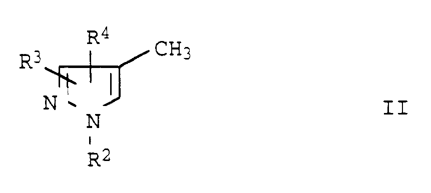 in Gegenwart eines Alkanols R¹-OH elektrochemisch oxidiert.

2. Verfahren nach Anspruch 1, dadurch gekennzeichnet, daß man die elektrochemische Oxidation in ungeteilten Durchflußzellen durchführt.

3. Verfahren nach Anspruch 1, dadurch gekennzeichnet, daß man die elektrochemische Oxidation an Graphitelektroden durchführt.

## Claims

1. A process for preparing 4-dialkoxymethylpyrazoles of the general formula I 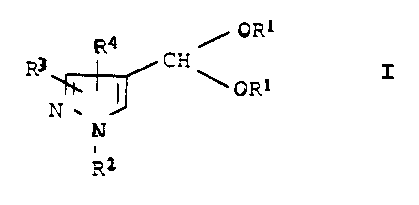 where
R¹ is n-alkyl,
R² is C₁-C₁₂-alkyl, C₃-C₁₂-cycloalkyl or phenyl, each of which may be substituted,
R³ and R⁴ are each hydrogen, C₃-C₁₂-cycloalkyl or phenyl, each of which may be substituted, cyano, halogen or alkoxycarbonyl,
which comprises electrochemical oxidation of a 4-methylpyrazole of the formula II 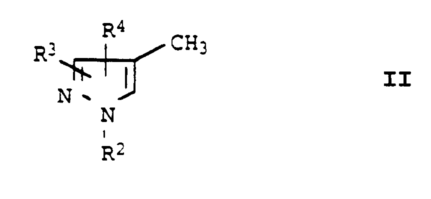 in the presence of an alkanol R¹-OH.

2. A process as claimed in claim 1, wherein the electrochemical oxidation is carried out in undivided flow cells.

3. A process as claimed in claim 1, wherein the electrochemical oxidation is carried out on graphite electrodes.

## Revendications

1. Procédé de préparation de 4-dialcoxyméthylpyrazoles de formule générale I 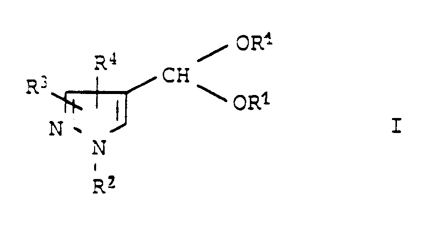 dans laquelle les substituants ont les significations suivantes:
R¹ Groupement n-alkyle,
R² Groupement alkyle en C₁-C₁₂, groupement cycloalkyle en C₃-C₁₂ ou groupement phényle, dont chacun peut être éventuellement substitué,
R³, R⁴ Atomes d'hydrogène, groupements cycloalkyle en C₃-C₁₂ ou groupements phényle, dont chacun peut être éventuellement substitué, groupements cyano, atomes d'halogène ou groupements alcoxycarbonyle,
caractérisé en ce que l'on oxyde par voie électrochimique un 4-méthylpyrazole de formule générale II 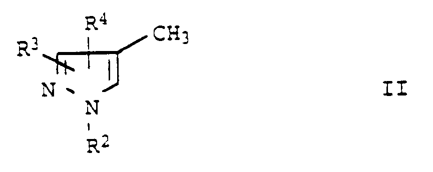 en présence d'un alcanol R¹-OH.

2. Procédé selon la revendication 1, caractérisé en ce que l'on conduit l'oxydation électrochimique dans des cellules à circulation non divisées.

3. Procédé selon la revendication 1, caractérisé en ce que l'on conduit l'oxydation électrochimique sur des électrodes en graphite.
